# EUROPEAN PATENT SPECIFICATION

(11) **EP 3 689 359 B1**
(45) Date of publication and mention of the grant of the patent: **20.09.2023**
(21) Application number: 20154741.1
(22) Date of filing: 30.01.2020
(51) Int. Cl.: A61K 36/28, A61P 31/16

(54) **ECHINACEA PALLIDA EXTRACT AND RELATING PROCESS OF PREPARATION AND PHARMACEUTICAL, COSMETIC AND NUTRACEUTIC COMPOSITIONS THEREOF**
ECHINACEA PALLIDA EXTRAKT, HERSTELLUNGSVERFAHREN, SOWIE PHARMAZEUTISCHE, KOSMETISCHE UND NUTRAZEUTISCHE ZUSAMMENSETZUNGEN
EXTRAIT DE RACINE D'ECHINACEA PALLIDA, PROCÉDÉ D'EXTRACTION ET COMPOSITIONS PHARMACEUTIQUES, COSMETIQUES ET NUTRACEUTIQUES

(30) Priority: 31.01.2019 IT 201900001405
(43) Date of publication of application: 05.08.2020
(73) Proprietor: Unifarco S.p.A., 32035 Santa Giustina (BL) (IT)
(72) Inventor: Baratto, Giovanni, 32035 Santa Giustina BL (IT)
(74) Representative: Perani & Partners S.p.A.

(56) References cited:
- WO-A1-2004/014404
- DALBY-BROWN LEA ET AL: "Synergistic antioxidative effects of alkamides, caffeic acid derivatives, and polysaccharide fractions from Echinacea purpurea on in vitro oxidation of human low-density lipoproteins", JOURNAL OF AGRICULTURAL AND FOOD CHEMISTRY, AMERICAN CHEMICAL SOCIETY, BOOKS AND JOURNALS DIVISION, US , vol. 53, no. 24 30 November 2005 (2005-11-30), pages 9413-9423, XP009516299, ISSN: 0021-8561, DOI: 10.1021/JF0502395 Retrieved from the Internet: URL:http://pubs.acs.org/doi/abs/10.1021/jf 0502395
- LI T S C: "EFFECTS OF ROOT DRYING TEMPERATURE AND MOISTURE CONTENT ON THE LEVELS OF ACTIVE INGREDIENTS IN ECHINACEA ROOTS", JOURNAL OF HERBS, SPICES & MEDICINAL PLANTS,, vol. 8, no. 1, 1 January 2001 (2001-01-01) , pages 15-22, XP009017220, ISSN: 1049-6475, DOI: 10.1300/J044V08N01_03
- BARNES J ET AL: "Echinacea species (Echinacea angustifolia (DC.) Hell., Echinacea pallida (Nutt.) Nutt., Echinacea purpurea (L.) Moench): A review of their chemistry, pharmacology and clinical properties", JOURNAL OF PHARMACY AND PHARMACOLOGY, JOHN WILEY & SONS LTD, LONDON, GB, vol. 57, no. 8, 1 August 2005 (2005-08-01) , pages 929-954, XP008112322, ISSN: 0022-3573, DOI: 10.1211/0022357056127

## Description

### FIELD OF THE INVENTION

The present invention relates to an *Echinacea pallida* extract with a very low alkylamide content for use in pharmaceutical, nutraceutical and food compositions.

### STATE OF THE ART

The genus *Echinacea* (Asteraceae) includes a small number of herbaceous and perennial hardy species native to North America. Three of these species, *Echinacea angustifolia*, *Echinacea pallida* and *Echinacea purpurea*, were considered different varieties of the same species. Now instead, following a taxonomic revision of the genus, this plant includes two subgenres and four species, namely *E. purpurea*, *E. pallida*, *E. atrorubens* and *Elaevigata*, whereby *E. angustifolia* and *E. pallida* have now been classified as *E. pallida var. angustifolia* and *E. pallida var. pallida.*

In any case, Joanne Barnes et al. in the review entitled *Echinacea species (Echinacea angustifolia* (DC) Hell: *Echinacea pallida* (Nutt.) Nut., *Echinacea purpurea* (L.) Moench): a review of their chemistry, pharmacology and clinical properties) describes them as three species: *E. angustifolia, E. pallida* and *E. purpurea.*

As reported in this review, *Echinacea* has long been used in the medicinal field, mainly in the field of infections such as syphilis and septic wounds, but also as an antitoxin for snake bites.

The current interest is mainly focused on its immunostimulating properties, in particular in the treatment and prevention of the common cold, flu and other upper respiratory tract infections, the so-called URTI.

The main constituents of the *Echinacea* genus are caffeoil derivatives (echinacoside, the chicoric, chlorogenic and kaftaric acids).

As far as alkamides or alkylamides are concerned, they are present both in *E*. *purpurea* and in *E. angustifolia*, whereas they are present in a decidedly lower amount in *E. pallida.*

Although alkamides show high immunomodulating properties via the cannabinoid type 2 receptor, however, due to their high lipophilicity and their tendency to accumulate in tissues, more in-depth studies are needed to test their effective non-toxicity.

As reported in the above review of Barnes et al., there are some differences in the constituents of each *Echinacea*, as better identified in the following table from the above review by Joanne Barnes et al.

**Table 1 Major constituents of Echinacea species used medicinally (adapted from Barnes 2002)**

| **Species** | **Plant part** | **Constituents** | **Comment** |
|---|---|---|---|
| *Echinacea purpurea* | Aerial parts | Alkamides; caffeic acid esters, mainly cichoric acid; polysaccharides; polyace tylenes | Echinacoside is not present |
| *Echinacea angustifolia* | Roots | Alkamides; caffeie acid esters, particularly echinacoside; cynarin; polysaccharides; polyacetylenes | Cynarin is characteristic of *E*. *angustifolia* |
| *Echinacea pallida* | Roots | Caffeic acid eaters, particulary echinacoside; polysaccharides; polyacetylenes (distinctive series) | Alkamides largely absent |

As it can be observed, for example, echinacoside is present in *E. angustifolia* and *E*. *pallida*, whereas it is absent in *E. purpurea*, whereas alkamides are instead contained in *E. pallida* in much lower quantities than in the other two species.

EP1539203 describes an extract of *E. angustifolia* roots having a lower content of 0.1% in alkylamides and a content of polysaccharides between 1 and 15%, and in which the polysaccharide has a molecular weight of 1.3×10⁵ Da and consists of rhamnose, arabinose, galactose and galacturonic acid in a specific ratio of 0.5:2.5:1.75:10.25.

Although the extract disclosed herein is an excellent product, intended for nutraceutical, pharmaceutical or even in the food field, it has a fair amount of alkylamides, whose drawbacks are reported above. Furthermore, the extraction process contemplates many washing steps with solvents that inevitably remain trapped, albeit in small quantities, in the final product.

The need is therefore felt to have an Echinacea extract, which does not have the aforementioned drawbacks and which at the same time can be produced with a less laborious process and in a purer form, allowing it to be used safely in the food field.

### SUMMARY OF THE INVENTION

The applicant has now found that it is possible to overcome the aforementioned problems of the state of the art with the extract according to the present invention. The object of the present invention is therefore an extract from *Echinacea pallida* roots.

It is described an *Echinacea pallida* extract having:
- an alkylamide content < 0.05% based on the total weight of the extract;
- an echinacoside content ≥ 2% by weight based on the total weight of the extract,
- a total content of polysaccharides ≥ 30% by weight based on the total weight of the extract,
- and a polysaccharide content with an average weight molecular weight higher than or equal to 12,000 Da ≥ 5%.

This extract is obtained with a process that includes the following steps:
a) extraction of the fraction containing echinacoside by hot mixing *Echinacea pallida* roots in hydroalcoholic solvent,
b) small volume concentration of the solvent of the mixture coming from the previous step until obtaining a soft extract with a concentration between 20° Bx and 40° Bx,
c) extraction of the fraction containing polysaccharides by mixing *Echinacea pallida* root in hot water,
d) small volume concentration of water until obtaining a soft extract with a concentration between 20° Bx and 40° Bx,
e) union of the two soft extracts obtained in steps b) and d) and drying by means of one of the methods known from the technical practice, preferably in a fluidized bed granulator containing maltodextrin and silica. A further object of the present invention is the use of said extract as a medicament, as a cosmetic or as a nutraceutical.

Finally, an object of the present invention are nutraceutical compositions, cosmetic compositions or pharmaceutical compositions containing as an active principle the extract object of the present invention in combination with suitable excipients and/or diluents.

### DESCRIPTION OF THE FIGURES

Figures 1 and 2 show the chromatograms of the fraction of the extract of echinacoside and alkylamides of the invention, which, as shown in Figure 2, are contained in said extract in quantities equal to 0.00381 ± 0.0005 (m/m%)
Figure 3 shows the chromatogram of the fraction of the extract containing polysaccharides.
Figure 4 shows the IL-1β concentrations measured in ng/ml expressed by macrophages isolated from buffy coat of human venous blood in the absence or at different concentrations of the *E. pallida* extract in the absence or presence of the inflammatory agent LPS.
Figure 5 shows the TNF-α concentrations measured in ng/ml expressed by macrophages isolated from buffy coat of human venous blood in the absence or at different concentrations of the *E. pallida* extract in the absence or presence of LPS.
Figure 6 shows IL-1β expressed by dendritic cells (monocytes) from buffy coat of human venous blood grown for 7 days in RPMI1640, in the presence or not of Echinacea.
Figure 7 shows TNF-α expressed by dendritic cells (monocytes) from buffy coat of venous human blood grown for 7 days in RPMI1640 in the presence or not of Echinacea.
Figure 8 shows the concentration of dendritic cells (monocytes) from buffy coat of human venous blood incubated in the presence or absence of Salmonella and in the presence or not of Echinacea subjected to CD80 staining (activation marker).
Figure 9 shows the concentration of the same cells at the end of the incubation subjected to staining with anti-CD11c (marker for dendritic cells).
Figure 10 shows the concentration of the same dendritic cells (monocytes) from buffy coat of human venous blood in the presence or not of Salmonella and in the presence or not of Echinacea subjected to flow cytometric analysis to determine the percentage of CD11c cells positive for the two activation markers (CD80 and HLA-DR graphs in Figures 10 and 11) and for the intensity of the fluorescent signal (MFI) indicative of the expression of each protein.
Figure 11 shows the concentration of the same type of cells subjected to HLA-DR (activation marker).

### DETAILED DESCRIPTION OF THE INVENTION

For the purposes of the present invention, the definitions "comprising" or "containing" do not exclude the presence of further components other than those described after this definition, which is very different from the definitions "constituted" or "consisting" that exclude the presence of further components described after these definitions.

For the purposes of the present invention, nutraceutical corresponds to the definition given by Stephan Defelice in 1989, namely a food (or part of it), preferably a part of it (specifically the extract object of the present invention), which has positive effects on well-being and health, including disease prevention and treatment.

Nutraceutical composition means an oral formulation containing said nutraceutical in combination with suitable excipients and/or diluents.

Pharmaceutical composition means a preferably oral formulation containing the extract object of the present invention as a pharmaceutical active ingredient in combination with suitable excipients and/or diluents.

Cosmetic composition means a preferably topical formulation containing the above extract as an active ingredient in combination with suitable excipients and/or diluents.

The echinacoside content is preferably between 2 and 5%, more preferably between 2 and 3% by weight based on the total weight of the extract. The alkylamide content is preferably between 0.001 and 0.05% based on the total weight of the extract.

The total polysaccharide content is preferably between 30 and 80% by weight, whereas the polysaccharide content with an average weight molecular weight higher than or equal to 12,000 Da is preferably between 5 and 12% by weight based on the total weight of the extract.

In the extract preparation process according to the present invention, the temperature at which step a) is carried out is preferably between 20 and 60°C, but also between 20 and 40°C. The hydroalcoholic solvent is preferably ethanol at a concentration of between 40 and 80%, preferably 80%.

In step b), the extract obtained is concentrated in a small volume until obtaining a soft extract, for example with a concentration of 31.5° Bx.

Step c) is preferably carried out at a temperature between 20 and 80°C, whereas in step d) it is concentrated, for example, up to 25.5° Bx.

In step e), for example, maltodextrin is 23% based on the total weight of the extract and silica is 5% based on the total weight of the extract.

The extract object of the present invention is used in particular in the treatment and prevention of the common cold, flu and other upper respiratory tract infections (URTI).

The following examples of preparation of the *Echinacea pallida* extract and of the relative tests of the immunomodulatory effect exerted by the *Echinacea pallida* extract according to the present invention are reported for illustrative purposes.

### Example 1 - Obtaining the soft extract enriched in echinacoside

267 g of *Echinacea pallida* root are placed in an extractor/mixer to which 3.2 kg of a hydroalcoholic solution (80% ethanol) are added and extracted for 4 hours.

Then the obtained extract is concentrated until obtaining a soft extract with a concentration of 31.5 Bx. 130 g of soft extract are obtained.

### Example 2 - Obtaining the soft extract in polysaccharides

Using approximately the same quantities of root and the same volume (water), the fraction rich in polysaccharides is extracted. After four hours it concentrates at a small volume up to 25.5 Bx. 125 g of soft extract are thus obtained.

### Example 3 - Mixing the soft extracts and drying

130 g of soft extract equal to 40.95 g of dry extract containing 4.92% of echinacoside are combined with 80.3 g of extract rich in polysaccharides corresponding to 20.48 g of dry extract in a fluidized bed dryer to which 23.7 g of maltodextrin and 6.8 g of silica are added. 80 g of extract containing 2% of echinacoside are obtained.

### Example 4 - Determining the polysaccharide content by HPLC

For the characterization of the extracts containing the polysaccharides, a Phenomenex Polysep -GFC-P3000 column (300 × 7.8 mm with Guard Column precolumn) is used, having as eluent buffer ammonium acetate 0.1M and acetonitrile (99:1) in isocratic conditions at a flow of 0.5 ml/min. During the 30-minute analysis, the column is kept at a temperature of 50°C.

The injection volume is 10 µl and an ELSD Sedex mod.60LT detector is coupled to the column described above. The detector's nebulizer is maintained at a temperature of 60°C, with a gas pressure of 2.3 bar.

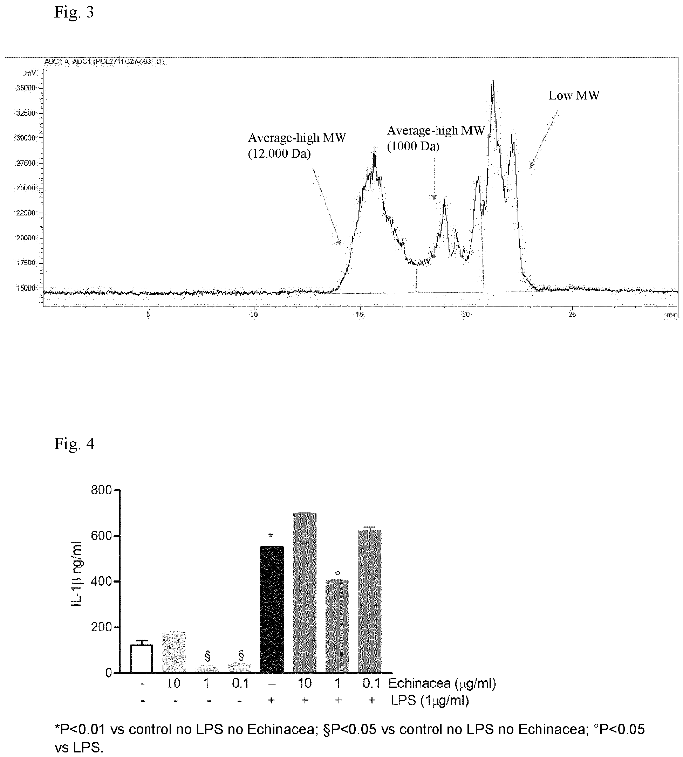

Figure 3 shows the chromatogram of the fraction containing the polysaccharides.

### Example 5 - Determining the content of echinacoside and alkylamides by HPLC

An Agilent SB C-18 reverse phase column (4.6 × 50 mm 1.8 µm, using acid water for elution (1% formic acid) and acetonitrile, at a flow of 1.0 ml/minute, is used for the determination of the content of echinacoside and alkylamides in the extract of *E*. *pallida* object of the invention. During the 7.5-minute analysis, the column is kept at room temperature. The injection volume is 10 µl, and the column described above is coupled with a UV-visible detector, the detection wavelength being 330 nm.

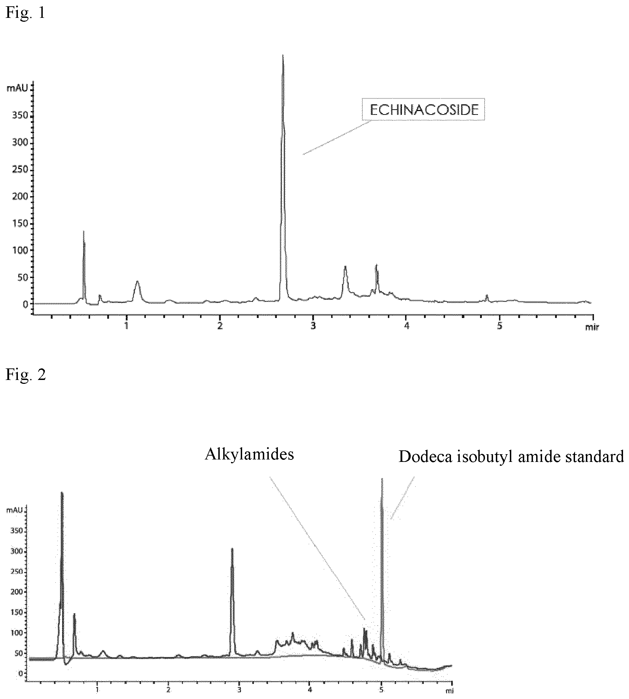

Figures 1 and 2 show the chromatograms of echinacoside and alkylamides, respectively.

### Example 6 - Evaluating the immunomodulatory activity of an Echinacea pallida extract

### Example 6-1 Anti-inflammatory activity

The anti-inflammatory activity was tested *in vitro* on differentiated macrophages starting from circulating human monocytes.

Macrophages were obtained by isolating monocytes from the human venous blood buffy coat. These were grown in RPMI 1640 containing 5% FCS, 1% antibiotics and rh M-CSF (10 ng/ml) for 10 days. The medium was replaced every 48 hours. On the day of the experiment, the cells were placed in fresh complete medium with or without LPS in the presence or absence of Echinacea extract at a concentration of 10 - 1 - 0.1 µg/ml. The medium was collected after 24 hours and immediately frozen. ELISA assays specific for IL-1β, TNF-α have been used for dosing the cytokines of interest. The obtained data are shown in Figures 4-5.

### Example 6-2 - Immuno-modulating activity

Immuno-modulating activity was tested *in vitro* on differentiated dendritic cells starting from circulating human monocytes by means of two types of measurements.

Dendritic cells were obtained by isolating monocytes from the human venous blood buffy coat. These were grown in RPMI 1640 containing 5% FCS, 1% antibiotics, rh GM-CSF and rh IL-4. (10 ng/ml each) for 7 days. The medium was replaced every 48 hours by centrifugation of the dendritic cells growing in suspension.

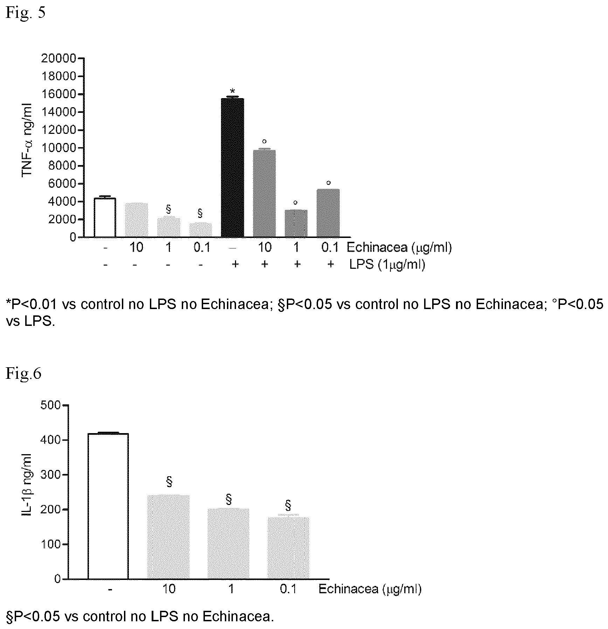

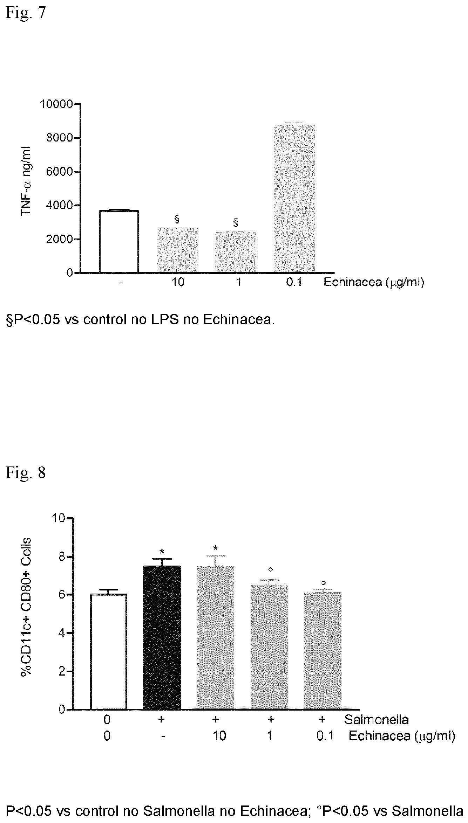

In a first series of assays, on the day of the experiment, the cells were placed in fresh complete medium with or without Echinacea extract at a concentration of 10 - 1 - 0.1 µg/ml. The medium was collected after 24 hours and immediately frozen. ELISA assays specific for IL-1β, TNF-α have been used for dosing the cytokines of interest. The obtained data are shown in the graphs of Figures 6 and 7.

In a second series of experiments, the dendritic cells were incubated for one hour with or without Salmonella (MOI 1:1) in antibiotic-free medium at 37°C. At the end of the incubation, the cells were collected by low speed centrifugation and washed twice with sterile RPMI to remove the bacteria that did not penetrate the cells. At the end, the dendritic cells were resuspended in complete fresh medium containing antibiotic with or without Echinacea extract at a concentration of 10 - 1 - 0.1 µg/ml.

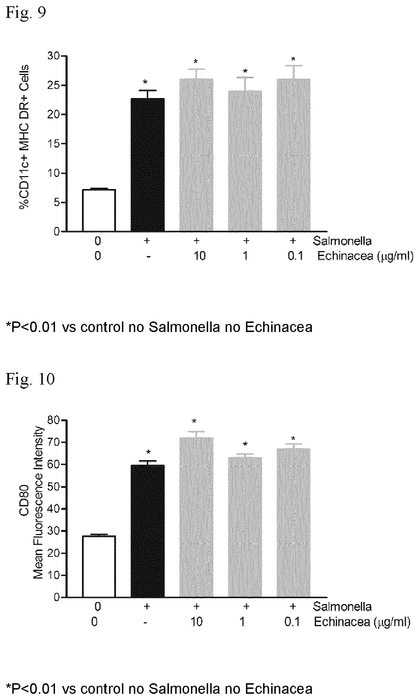

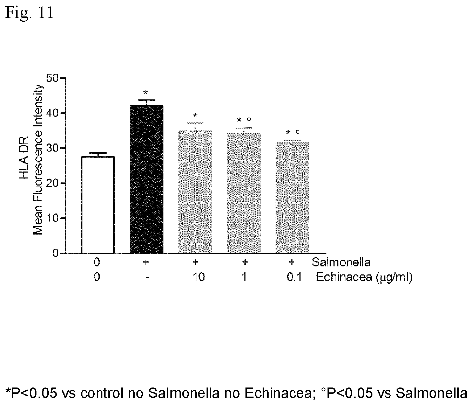

Then the cells were incubated for 24 h at 37°C. At the end of the incubation, they were collected by centrifugation and stained with anti-CD11c (marker for dendritic cells) and CD80 or HLA-DR (activation markers). Then the cells were subjected to flow cytometric analysis to determine the percentage of CD11c cells positive for the two activation markers and for the intensity of the fluorescent signal (MFI) indicative of the expression of each protein. The obtained data are shown in the following graphs of Figures 8-11.

### Example 6-3 - Conclusions

The assays performed with human macrophages show a good anti-inflammatory activity, evidenced by the ability of the Echinacea extract to significantly inhibit the production of two powerful pro-inflammatory cytokines (IL-1β and TNFα). The effects appear more consistent at the dose of 1 µg/ml of extract. Higher doses seem to cause, at least *in vitro*, the nonspecific activation of macrophages, whereas the dose of 0.1 µg/ml gives reasonable results: on non-activated macrophages it is able to reduce the basal production of IL-1β and TNFα, whereas on cells activated by the LPS it has no effect. Overall, the "immuno-modulating" activity, assessed as the ability to influence the phenotype and the response of human dendritic cells to a powerful pro-inflammatory stimulus, seems less energetic than the anti-inflammatory one, albeit present. The extract significantly and consistently reduces the production of pro-inflammatory cytokines from dendritic cells under basal conditions at a dose of 1 µg/ml and reduces the expression of CD80 and MHC II (in one case by reducing the percentage of positive cells, in the other by reducing the quantity of molecules expressed per cell).

In conclusion, the preliminary *in vitro* assays on macrophages and human dendritic cells have shown excellent anti-inflammatory activity and a moderate immuno-modulating action of the tested Echinacea extract.

## Claims

1. Extract from *Echinacea pallida* roots having:
• a content of alkylamides < 0.05%
• a content of echinacoside ≥ 2%,
• a total content of polysaccharides ≥ 30%
• and a content of polysaccharides with a weight average molecular weight higher than 12,000 Da, ≥ 5%.

2. The extract according to claim 1, wherein the content of alkylamides is between 0.001 and 0.05%.

3. The extract according to claim 1 or 2, wherein the content of echinacoside is comprised between 2 and 5%, preferably between 2 and 3% by weight based on the total weight of the extract.

4. The extract according to any one of claims 1-3, wherein the total polysaccharide content is between 30 and 80% by weight based on the total weight of the extract.

5. The extract according to any one of claims 1-4, wherein the content of polysaccharides with a weight average molecular weight is between 5 and 12% by weight based on the total weight of the extract.

6. Process for preparing the extract according to any one of claims 1-5, comprising the following steps:
a) extraction of the fraction containing echinacoside by hot mixing *Echinacea pallida* roots in a hydroalcoholic solvent,
b) concentration to a small volume of the solvent of the mixture from the previous step until obtaining a soft extract with a solid component concentration between 20 and 40° Bx,
c) extraction of the fraction containing polysaccharides by mixing in hot water the *Echinacea pallida* root,
d) concentration to a small volume of water until obtaining a soft extract with a solid component concentration between 20 and 40° Bx,
e) union of the two soft extracts obtained in steps b) and d) and drying in a fluid bed granulator containing maltodextrin and silica.

7. The process according to claim 6, wherein the temperature of step a) is between 20 and 40°C and said hydroalcoholic solvent is preferably ethanol at a concentration ranging from 40 to 80%.

8. The process according to claim 6, wherein step a) is carried out at a temperature comprised between 20 and 60°C and said hydroalcoholic solvent is ethanol at a concentration ranging from 40 to 80%.

9. The process according to any one of the claims 6-8, wherein step c) is carried out at a temperature ranging from 20 to 80°C.

10. The extract according to any one of the claims 1-5 for use as a nutraceutical, as a cosmetic or as a medicament.

11. The extract according to any one of the claims 1-5 for use in the treatment and prevention of the common cold, flu and other respiratory tract infections (URTI).

12. Pharmaceutical, cosmetic or nutraceutical compositions containing as the active ingredient the extract according to any one of the claims 1-5 in combination with suitable excipients and/or diluents.

13. The pharmaceutical compositions according to claim 12, suitable for oral administration.

## Patentansprüche

1. Extrakt aus *Echinacea pallida*-Wurzeln aufweisend:
• einen Gehalt an Alkylamiden < 0,05 %
• einen Gehalt an Echinacosid > 2 %,
• einen Gesamtgehalt an Polysacchariden > 30 %
• und einen Gehalt an Polysacchariden mit einem gewichtsmittleren Molekulargewicht von mehr als 12.000 Da, > 5 %.

2. Extrakt nach Anspruch 1, wobei der Gehalt an Alkylamiden zwischen 0,001 und 0,05 % liegt.

3. Extrakt nach Anspruch 1 oder 2, wobei der Gehalt an Echinacosid zwischen 2 und 5 Gew.-%, vorzugsweise zwischen 2 und 3 Gew.-%, bezogen auf das Gesamtgewicht des Extrakts, beträgt.

4. Extrakt nach einem der Ansprüche 1 bis 3, wobei der Gesamtpolysaccharidgehalt zwischen 30 und 80 Gew.-%, bezogen auf das Gesamtgewicht des Extrakts, beträgt.

5. Extrakt nach einem der Ansprüche 1 bis 4, wobei der Gehalt an Polysacchariden mit einem gewichtsmittleren Molekulargewicht zwischen 5 und 12 Gew.-%, bezogen auf das Gesamtgewicht des Extrakts, beträgt.

6. Verfahren zur Herstellung des Extrakts nach einem der Ansprüche 1 bis 5, umfassend die folgenden Schritte:
a) Extraktion der echinacosidhaltigen Fraktion durch Heißmischen von *Echinacea pallida-*Wurzeln in einem hydroalkoholischen Lösungsmittel,
b) Konzentration auf ein kleines Volumen des Lösungsmittels der Mischung aus dem vorhergehenden Schritt, bis ein weicher Extrakt mit einer Feststoffkonzentration zwischen 20 und 40° Bx erhalten wird,
c) Extraktion der polysaccharidhaltigen Fraktion durch Mischen in heißem Wasser der *Echinacea pallida-*Wurzel,
d) Konzentration auf ein kleines Wasservolumen, bis ein weicher Extrakt mit einer Feststoffkonzentration zwischen 20 und 40° Bx erhalten wird,
e) Vereinen der beiden in den Schritten b) und d) erhaltenen weichen Extrakte und Trocknung in einem Wirbelschichtgranulator, der Maltodextrin und Kieselsäure enthält.

7. Verfahren nach Anspruch 6, wobei die Temperatur von Schritt a) zwischen 20 und 40 °C liegt und das hydroalkoholische Lösungsmittel vorzugsweise Ethanol in einer Konzentration im Bereich von 40 bis 80 % ist.

8. Verfahren nach Anspruch 6, wobei Schritt a) bei einer Temperatur zwischen 20 und 60 °C durchgeführt wird und das hydroalkoholische Lösungsmittel Ethanol in einer Konzentration im Bereich von 40 bis 80 % ist.

9. Verfahren nach einem der Ansprüche 6 bis 8, wobei Schritt c) bei einer Temperatur im Bereich von 20 bis 80 °C durchgeführt wird.

10. Extrakt nach einem der Ansprüche 1 bis 5 zur Verwendung als Nutrazeutikum, als Kosmetikum oder als Medikament.

11. Extrakt nach einem der Ansprüche 1 bis 5 zur Verwendung bei der Behandlung und Vorbeugung von Erkältung, Grippe und anderen Atemwegsinfektionen (URTI).

12. Pharmazeutische, kosmetische oder nutrazeutische Zusammensetzungen, die als Wirkstoff den Extrakt nach einem der Ansprüche 1 bis 5 in Kombination mit geeigneten Exzipienten und/oder Verdünnungsmitteln enthalten.

13. Pharmazeutische Zusammensetzungen nach Anspruch 12, die zur oralen Verabreichung geeignet sind.

## Revendications

1. Extrait de racines *d'Echinacea pallida* ayant :
• une teneur en alkylamides < 0,05%
• une teneur en échinacoside > 2%,
• une teneur totale en polysaccharides > 30 %
• et une teneur en polysaccharides avec une masse moléculaire moyenne en poids supérieure à 12 000 Da, > 5 %.

2. Extrait selon la revendication 1, dans lequel la teneur en alkylamides est comprise entre 0,001 et 0,05 %.

3. Extrait selon la revendication 1 ou 2, dans lequel la teneur en échinacoside est comprise entre 2 et 5 %, de préférence entre 2 et 3 % en poids par rapport au poids total de l'extrait.

4. Extrait selon l'une quelconque des revendications 1 à 3, dans lequel la teneur totale en polysaccharides est comprise entre 30 et 80% en poids par rapport au poids total de l'extrait.

5. Extrait selon l'une quelconque des revendications 1 à 4, dans lequel la teneur en polysaccharides avec une masse moléculaire moyenne en poids est comprise entre 5 et 12 % en poids par rapport au poids total de l'extrait.

6. Procédé de préparation de l'extrait selon l'une quelconque des revendications 1 à 5, comprenant les étapes suivantes :
a) extraction de la fraction contenant de l'échinacoside par mélange à chaud des racines d'*Echinacea pallida* dans un solvant hydro-alcoolique,
b) concentration à un petit volume du solvant du mélange provenant de l'étape précédente jusqu'à l'obtention d'un extrait mou avec une concentration en composants solides comprise entre 20 et 40° Bx,
c) extraction de la fraction contenant des polysaccharides en mélangeant dans de l'eau chaude la racine d'*Echinacea pallida*,
d) concentration à un petit volume d'eau jusqu'à l'obtention d'un extrait mou avec une concentration en composants solides comprise entre 20 et 40° Bx,
e) union des deux extraits mous obtenus dans les étapes b) et d) et séchage dans un granulateur à lit fluidisé contenant de la maltodextrine et de la silice.

7. Procédé selon la revendication 6, dans lequel la température de l'étape a) est comprise entre 20 et 40 °C et ledit solvant hydro-alcoolique est de préférence de l'éthanol à une concentration allant de 40 à 80 %.

8. Procédé selon la revendication 6, dans lequel l'étape a) est réalisée à une température comprise entre 20 et 60°C et ledit solvant hydro-alcoolique est de l'éthanol à une concentration allant de 40 à 80 %.

9. Procédé selon l'une quelconque des revendications 6 à 8, dans lequel l'étape c) est réalisée à une température allant de 20 à 80°C.

10. Extrait selon l'une quelconque des revendications 1 à 5, destiné à être utilisé comme nutraceutique, comme cosmétique ou comme médicament.

11. Extrait selon l'une quelconque des revendications 1 à 5, destiné à être utilisé dans le traitement et la prévention du rhume, de la grippe et d'autres infections des voies respiratoires (IVRS).

12. Compositions pharmaceutiques, cosmétiques ou nutraceutiques contenant comme ingrédient actif l'extrait selon l'une quelconque des revendications 1 à 5 en combinaison avec des excipients et/ou diluants appropriés.

13. Compositions pharmaceutiques selon la revendication 12, appropriées pour l'administration orale.
